# FASCICULE DE BREVET EUROPEEN

(11) **EP 3 558 108 B1**
(45) Date de publication et mention de la délivrance du brevet: **26.08.2026**
(21) Numéro de dépôt: 17832300.2
(22) Date de dépôt: 22.12.2017
(51) Int. Cl.: A61B 5/00, A61B 5/25, A61B 5/053, H01M 4/74, A61N 1/04, D02G 3/44

(54) **INDICATEUR DE PERTURBATIONS POUR UN DISPOSITIF DESTINÉ À ÊTRE PORTÉ**
STÖRUNGSANZEIGE FÜR EINE WEARABLE-VORRICHTUNG
DISTURBANCES INDICATOR FOR A WEARABLE DEVICE

(30) Priorité: 26.12.2016 FR 1663387
(43) Date de publication de la demande: 30.10.2019
(73) Titulaire: Bioserenity Medical Devices Group, 75013 Paris (FR)
(72) Inventeur: PROT, Pierre, 75013 Paris (FR); FROUIN, Pierre-Yves, 75013 Paris (FR)
(74) Mandataire: Icosa
(86) Numéro de dépôt international: PCT/FR2017/053832
(87) Numéro de publication internationale: WO 2018/122522

(56) Documents cités:
- EP-A1- 2 407 096
- EP-A1- 2 407 096
- EP-B1- 0 855 875
- US-A1- 2015 015 452
- US-A1- 2015 015 452
- US-A1- 2016 022 216
- US-A1- 2016 022 216
- TAE-HO KANG: "Textile -embedded sensors for wearable physiological monitoring systems", 19 May 2007 (2007-05-19), pages 1 - 144, XP055402313, ISBN: 978-0-549-20213-4, Retrieved from the Internet <URL:https://repository.lib.ncsu.edu/bitstream/handle/1840.16/5143/etd.pdf?sequence=1&isAllowed=y> [retrieved on 20170830]
- TAE-HO KANG: "Textile -embedded sensors for wearable physiological monitoring systems", 19 May 2007 (2007-05-19), pages 1 - 144, XP055402313, ISBN: 978-0-549-20213-4, Retrieved from the Internet <URL:https://repository.lib.ncsu.edu/bitstream/handle/1840.16/5143/etd.pdf?sequence=1&isAllowed=y> [retrieved on 20170830]

## Description

### DOMAINE DE L'INVENTION

La présente invention concerne un système de mesure des perturbations électromagnétiques ou électrostatiques destiné à être porté par un utilisateur. La présente invention concerne également un vêtement comprenant un tel système,

### ÉTAT DE LA TECHNIQUE

Les vêtements intelligents permettant de réaliser des mesures de signaux bioélectriques nécessitent de faire transiter des signaux de très faibles niveaux électriques (de l'ordre de quelques microvolts à quelques millivolts), par des moyens de connexions électriques de longueur non négligeable, pilotés par des impédances de contact élevées. Ces signaux sont alors soumis à un risque important de perturbations électromagnétiques ou électrostatiques. Ces perturbations peuvent être variées. Elles peuvent être causées par des ondes hertziennes de Haute Fréquence, des perturbations de type électrostatiques ou de Basse Fréquence, par exemple si une autre personne passe à proximité des capteurs. La tension du secteur électrique est également susceptible de générer du bruit à 50 Hz ou 60 Hz sur les signaux mesurés et transmis.

Ces perturbations gênent l'exploitation et l'interprétation des signaux, qu'elles soient faites manuellement par les techniciens ou les médecins ; ou informatiquement par des algorithmes. Dans le domaine de la médecine, l'expertise du médecin peut lui permettre de distinguer différents types d'artefacts mais de façon très limitée. Ces perturbations électromagnétiques ou électrostatiques peuvent aller jusqu'à fausser un diagnostic ou l'émettre avec des réserves.

Le document US 2009/318827 décrit un système et une méthode pour monitorer l'activité électrique du cerveau d'un sujet par le biais d'une pluralité d'électrodes placés en proximité d'une portion du corps du sujet. Ledit système comprend en outre une antenne.

L'antenne du document US 2009/318827 est apte à émettre des signaux mais n'est pas adaptée à la mesure de signaux électromagnétiques.

John et al. décrit un système comprenant un implant vasculaire, un capteur de pression et une antenne de transmission sans fil de données enregistrées par le capteur à une unité externe d'analyse (Biomedical Engineering, 2016, pp. 155-159). L'antenne décrite est apte à émettre des signaux mais n'est pas adaptée à la mesure de signaux électromagnétiques.

Kang et al. décrit un système pour l'acquisition des signaux physiologiques comprenant un circuit ayant plusieurs pistes conductrices et éléments électroniques qui visent à réduire l'impédance de sortie d'une électrode connectée audit circuit. Les pistes conductrices ne sont pas adaptées à la mesure de signaux électromagnétiques.

Le document EP 2 407 096 décrit une électrode textile pour la mesure de signaux du corps comprenant dans laquelle une piste conductrice est utilisée pour simplifier la connexion entre l'électrode et le système d'acquisition. La piste conductrice décrite n'est pas adaptée à la mesure de signaux électromagnétiques.

L'objet de la présente invention est donc de fournir un système de mesure des perturbations électromagnétiques ou électrostatiques destiné à être porté par un utilisateur qui puisse s'intégrer dans un vêtement. La présente invention permet ainsi de déterminer si les mesures réalisées sur un vêtement intelligent sont altérées par des perturbations de type électromagnétiques ou électrostatiques externes.

### RÉSUMÉ

La présente invention concerne un système de mesure des perturbations destiné à être porté par un utilisateur. Ledit système comprend au moins un moyen de mesure bioélectrique ; un dispositif de conversion analogique-numérique connecté électriquement audit au moins un moyen de mesure bioélectrique ; et au moins une piste conductrice connectée électriquement à une masse du système par l'intermédiaire d'une résistance et à une entrée du dispositif de conversion analogique-numérique.

Dans un mode de réalisation de l'invention, la longueur de ladite au moins une piste conductrice est supérieure à 1 cm. Dans un mode de réalisation de l'invention, la longueur de ladite au moins une piste conductrice est supérieure à 2 cm, 3 cm, 4 cm, 5 cm, 6 cm, 7 cm, 8 cm, 9 cm, 10 cm ou 20 cm, 30 cm, 40 cm ou 50 cm.

Dans un mode de réalisation de l'invention, le signal électrique mesuré par la au moins une piste conductrice est soustrait au signal électrique mesuré par le au moins un moyen de mesure bioélectrique pour produire un signal résultant épuré.

Dans un mode de réalisation de l'invention, une résistance couplée à la piste conductrice simule une impédance de contact entre la peau et au moins un moyen de mesure bioélectrique.

Dans un mode de réalisation de l'invention, l'impédance de ladite résistance est comprise entre 10 kOhms et 100 MOhms. Dans un mode de réalisation de l'invention, le système comprend en outre un condensateur connecté en parallèle de ladite résistance. Dans un mode de réalisation de l'invention, la capacité dudit condensateur est comprise entre de 10 picofarad et 100 nanofarad.

Dans un mode de réalisation de l'invention, la au moins une piste conductrice comprend une première extrémité connectée électriquement à la masse du système et à une entrée du dispositif de conversion analogique-numérique, et une deuxième extrémité libre. Dans un mode de réalisation de l'invention, la au moins une piste conductrice comprend une première extrémité connectée électriquement à la masse du système et une deuxième extrémité connectée électriquement à une entrée du dispositif de conversion analogique-numérique.

Ledit système comprend en outre un substrat textile dans lequel ledit moyen de mesure biométrique et ladite au moins une piste conductrice sont connectés mécaniquement audit substrat textile Dans un mode de réalisation de l'invention, le substrat est un substrat textile et la au moins une piste conductrice :
- comprend au moins un fil conducteur tissé, brodé, tricoté ou inséré à travers le substrat textile ; ou
- est une encre conductrice ou une peinture conductrice imprimée sur le substrat textile.

L'invention concerne également un vêtement comprenant au moins un système de mesure des perturbations selon la présente invention.

L'invention concerne également une méthode d'utilisation du système de mesure des perturbations selon la présente invention ou du vêtement selon la présente invention comprenant les étapes suivantes :
- la mesure du signal obtenu par le au moins un moyen de mesure bioélectrique :
- la mesure du signal obtenu par la au moins une piste conductrice ; et
- le traitement desdits signaux de manière à retirer, du signal mesuré par le au moins un moyen de mesure bioélectrique, le signal mesuré par la au moins une piste conductrice,

### DÉFINITIONS

Dans la présente invention, les termes ci-dessous sont définis de la manière suivante :
- Par « **Vêtement** » on entend tout textile apte à être porté par un sujet, y compris un bonnet.
- Par « **Antenne boucle** » on entend une antenne permettant de mesurer le champ magnétique de son environnement. Son principe de fonctionnement résulte d'une application de la loi de Lenz-Faraday, la tension induite étant proportionnelle au flux du champ magnétique.
- Par « **Dispositif destiné à être porté par un utilisateur** » on entend tout vêtement, habit, bonnet, sous-vêtement, apte à être porté par un utilisateur, de préférence au contact de la peau.
- Par « **Moyen de mesure bioélectrique** » on entend tout capteur apte à mesurer un signal électrique produit par un organisme vivant, au contact ou à distance de la peau.
- Par « **Dispositif de conversion analogique-numérique** » on entend un dispositif électronique qui transforme un signal électrique en données numériques.
- Par « **Piste conductrice** » on entend une zone conductrice. Cette piste conductrice peut consister en un ou plusieurs fils conducteurs, lesdits fils conducteurs sont constitués d'un matériau conducteur ou de fils textiles recouverts d'une surface conductrice ou d'un matériau conducteur, préférentiellement de fils textiles recouverts de métaux conducteurs comme de l'argent. Cette piste conductrice peut également consister en une encre conductrice ou une peinture conductrice sur un substrat, l'encre ou la peinture conductrice est chargée avec un matériau électriquement conducteur, possédant des propriétés de souplesse, permettant à cette encre ou cette peinture conductrice d'être déposée sur des surfaces souples.
- Par « **Extrémité** » on entend la fin de la partie extrême dans le sens de la plus grande des dimensions de la piste conductrice. Dans le cas où la piste conductrice est une surface, l'extrémité est constituée par la (ou les) avancée destinée à être connectée à une masse du système et/ou à une entrée d'un dispositif de conversion analogique-numérique.
- Par « **Substrat** » on entend une pièce servant de support à au moins un ou à la totalité des constituants du système selon la présente invention,
- Par « **Substrat textile** » on entend un substrat constitué de fils textiles isolants qui peuvent être tissés ou tricotés.
- Par « **Substrat mécanique** » on entend un substrat constitué d'au moins une pièce rigide, ou une pièce qui n'est pas un textile.
- Par « **Flexible** », on entend la capacité d'être courbé sur une pièce cylindrique ayant un rayon de 5 à 10 cm sans subir de déformation plastique.
- Lorsqu'une plage de valeurs est évoquée par l'expression « **compris entre A et B** », on entend que les deux valeurs citées « A » et « B » sont inclus dans la plage de valeurs évoquée. Sont également inclus toutes les valeurs à plus ou moins 10% des bornes de la plage de valeurs.

### DESCRIPTION DÉTAILLÉE

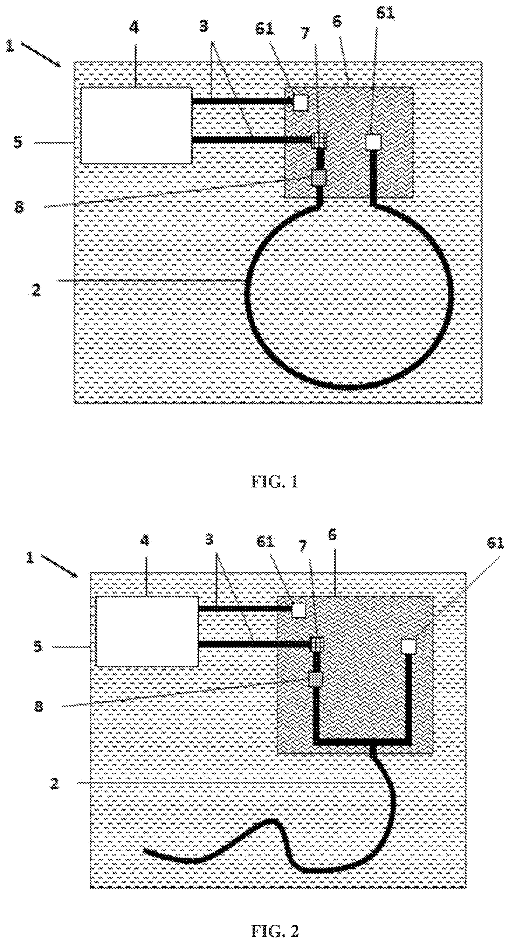

Comme illustré sur la **figure 1****,** la présente invention concerne un système de mesure de perturbations 1 destiné à être porté par un utilisateur. Ce système 1 comprend au moins un moyen de mesure bioélectrique 4 ; un dispositif de conversion analogique-numérique 6 connecté électriquement audit au moins un moyen de mesure bioélectrique 4 ; et au moins une piste conductrice 2 connectée électriquement à la masse 7 du système 1 par l'intermédiaire d'une résistance 8 et à une entrée 61 du dispositif de conversion analogique-numérique 6,

L'invention concerne un système de mesure des perturbations (1) destiné à être porté par un utilisateur, ledit système comprenant :
- au moins un moyen de mesure bioélectrique (4) pour mesurer un signal électrique corporel et susceptible de recevoir des perturbations électromagnétiques et/ou électrostatiques extérieures ;
- un dispositif de conversion analogique-numérique (6) connecté électriquement audit au moins un moyen de mesure bioélectrique (4) ; et
- au moins une piste conductrice (2) de mesure de signaux électromagnétiques et/ou électrostatiques, ladite piste conductrice (2) étant connectée électriquement à une masse (7) du système (1) par l'intermédiaire d'une résistance (8) et à une entrée (61) du dispositif de conversion analogique-numérique (6).

Le au moins un moyen de mesure bioélectrique 4 permet de mesurer des signaux électriques émis par le corps humain ou animal. Dans un mode de réalisation, ce moyen est une électrode métallique ou conductrice au contact de la peau capable de mesurer les très faibles variations de signaux électriques, comme ceux mesurés par électroencéphalographie (EEG), électrocardiographie (ECG) ou électromyographie (EMG). Dans un mode de réalisation, le moyen de mesure peut être également un capteur d'électrooculogramme (EOG) ou un moyen de détection des stimuli lumineux,

Selon la présente invention, le au moins un moyen de mesure bioélectrique 4 est connecté électriquement au dispositif de conversion analogique-numérique 6 par une connexion électrique 3. Ledit dispositif de conversion analogique-numérique 6 est capable de convertir en grandeur numérique le signal électrique provenant du au moins un moyen de mesure bioélectrique 4.

La piste conductrice 2 selon la présente invention joue le rôle d'une antenne. Elle doit donc s'étendre sur une longueur ou une surface suffisante pour détecter les perturbations électromagnétiques et/ou électrostatiques qui sont également enregistrées par le au moins un moyen de mesure bioélectrique 4 et qui perturbe le signal mesuré. La longueur ou la surface de cette piste conductrice 2 doit donc être suffisamment grande et son intérêt va au-delà d'une simple connexion électrique entre deux composants.

La piste conductrice 2 est connectée électriquement à la masse 7 du système 1 et à une entrée 61 du dispositif de conversion analogique-numérique 6. De cette manière, le dispositif de conversion analogique-numérique 6 est apte à convertir en grandeur numérique le signal électrique provenant de la piste conductrice 2.

Dans un mode de réalisation, la piste conductrice 2 est un chemin conducteur dans lequel deux dimensions ont une distance négligeable par rapport à la distance de la troisième dimension.

Dans un mode de réalisation illustré sur la **figure 1****,** ladite piste conductrice 2 est connectée électriquement à la masse 7 du système 1 par une première extrémité et est connectée électriquement à une entrée 61 du dispositif de conversion analogique numérique 6 par une deuxième extrémité. Dans un mode de réalisation, la piste conductrice 2 comprend une première extrémité connectée électriquement à la masse 7 du système 1 et une deuxième extrémité connectée électriquement à une entrée 61 du dispositif de conversion analogique-numérique 6.

Dans un mode de réalisation alternatif illustré **figure 2****,** ladite piste conductrice 2 comprend au moins deux extrémités, la première extrémité est connectée électriquement à la masse 7 du système 1 et à une entrée 61 du dispositif de conversion analogique-numérique 6 et la deuxième extrémité est libre. Dans ce mode de réalisation, la piste conductrice comprend une première extrémité connectée électriquement à la masse 7 du système 1 et à une entrée 61 du dispositif de conversion analogique-numérique 6, et une deuxième extrémité libre. Par « la deuxième extrémité est libre », on entend que cette extrémité n'est reliée à aucune résistance ou aucun élément électriquement conducteur, de manière à créer un circuit ouvert. Dans un mode de réalisation, la première extrémité est connectée à une entrée d'un circuit imprimé et ladite entrée du circuit imprimé est connectée électriquement à la masse du système 7 et à une entrée 61 du dispositif de conversion analogique-numérique 6.

Dans un mode de réalisation non représenté, la piste conductrice 2 comprend une pluralité d'extrémités libres.

La piste conductrice 2 doit avoir une longueur suffisamment grande pour permettre la mesure des perturbations, La piste conductrice 2 a une longueur supérieure à 1 cm, 2 cm, 3 cm, 4 cm, 5 cm, 6 cm, 7 cm, 8 cm, 9 cm, 10 cm, 20 cm, 30 cm, 40 cm ou 50 cm. La a piste conductrice 2 a une longueur comprise entre 5 cm et 500 cm, ou comprise entre 5 cm et 200 cm. Dans un mode de réalisation, la piste conductrice 2 a une longueur comprise entre 5 cm et 100 cm. Dans un mode de réalisation, la longueur maximale de la piste conductrice 2 n'est pas bornée.

Cette longueur permet une meilleure précision dans la mesure des perturbations, par exemple des perturbations électromagnétiques ou électrostatiques qui ne proviennent pas du corps de l'utilisateur portant le système 1 selon la présente invention.

En effet, la piste conductrice 2 doit avoir une longueur suffisante pour enregistrer les perturbations.

Dans un mode de réalisation alternatif non représenté, le rôle d'antenne est réalisé par une surface entourée d'une piste conductrice 2 connectée électriquement au dispositif de conversion analogique-numérique 6 et à une masse du système 7. La piste conductrice 2 doit alors entourer une surface suffisamment grande pour permettre la mesure des perturbations. Dans ce mode de réalisation, la surface entourée par la piste conductrice 2 a une surface supérieure à 1 cm². Dans un mode de réalisation, la surface entourée par la piste conductrice 2 est une surface supérieure à 10, 20, 30, 40, 50, 100, 200 ou 300 cm².

Dans un mode de réalisation, la piste conductrice 2 s'étend sur un substrat 5 textile ou mécanique.

Dans un mode de réalisation où le substrat 5 est un substrat 5 textile, la piste conductrice 2 est réalisée de manière à avoir la même souplesse que le substrat 5 textile. La piste conductrice 2 doit pouvoir être pliée sur elle-même sans subir de déformation plastique et sans subir de détériorations pouvant amener à une coupure de la connexion électrique, Dans un mode de réalisation, la piste conductrice 2 a une flexibilité lui permettant d'être courbée sur une pièce cylindrique ayant un rayon de 5 à 10 cm sans subir de déformation plastique.

Dans un mode de réalisation, la piste conductrice 2 forme une antenne boucle. Dans ce même mode de réalisation, la forme dessinée par la piste conductrice 2 peut être une forme ovale, circulaire, rectangulaire, triangulaire, une forme ellipsoïdale, ou toute autre forme.

Dans un mode de réalisation, le moyen de mesure bioélectrique 4 peut être situé à l'intérieure de la forme dessinée par la piste conductrice 2. Dans un mode de réalisation, le moyen de mesure bioélectrique 4 peut être situé sensiblement au centre de la forme dessinée par la piste conductrice 2. Le placement du moyen de mesure bioélectrique 4 à l'intérieur de la forme dessinée par la piste conductrice 2 permet une meilleure indication des perturbations électromagnétiques et/ou électrostatiques mesurées par le au moins un moyen de mesure bioélectrique 4 puisque ces perturbations vont être mesurées au plus près du au moins un moyen de mesure bioélectrique 4.

Dans un mode de réalisation, le signal électrique mesuré par la au moins une piste conductrice (2) est soustrait au signal électrique mesuré par le au moins un moyen de mesure bioélectrique (4) pour produire un signal résultant épuré.

Selon la présente invention, le dispositif de conversion analogique-numérique 6 est apte à convertir en grandeur numérique le signal électrique provenant de la piste conductrice 2 et le signal électrique provenant du au moins un moyen de mesure bioélectrique 4.

Les valeurs numériques issues de la piste conductrice 2 correspondent à des perturbations électromagnétiques et/ou électrostatiques également enregistrées par le au moins un moyen de mesure bioélectrique 4. En soustrayant les valeurs issues de la piste conductrice 2 des valeurs issues du au moins un moyen de mesure bioélectrique 4, on obtient les valeurs d'un signal épuré des signaux extérieurs parasites.

Dans un mode de réalisation particulier, le système 1 comprend également un moyen, (tel qu'un dispositif électronique ou un algorithme), permettant de soustraire des valeurs issues du au moins un moyen de mesure bioélectrique 4, les valeurs issues de la piste conductrices. Dans un autre mode de réalisation, cette opération peut être faite a posteriori, ou plus simplement, la personne chargée d'interpréter les mesures peut comparer les deux valeurs afin de faciliter son interprétation des valeurs issues du au moins un moyen de mesure bioélectrique 4.

Dans un mode de réalisation non représenté, le système 1 comprend également un circuit imprimé et le dispositif de conversion analogique-numérique 6 est intégré sur ce circuit imprimé.

Selon la présente invention, la au moins une piste conductrice 2 est reliée ou connectée électriquement à la masse du système 7 par l'intermédiaire d'une résistance 8. Cette résistance 8 permet d'opérer la mesure du signal électrique provenant de la piste conductrice 2. Les faibles fréquences ne pourraient en effet pas être mesurées sans résistance.

Dans un mode de réalisation, l'impédance de la résistance 8 doit être de même ordre de grandeur, et préférentiellement la plus proche possible de l'impédance de contact entre la peau et le au moins un moyen de mesure bioélectrique 4. Ainsi, les signaux électriques mesurés par la piste conductrice 2 peuvent être comparés aux signaux mesurés par le au moins un moyen de mesure bioélectrique 4.

Dans un mode de réalisation, la piste conductrice (2) est configurée pour recevoir des signaux tels que des signaux électromagnétiques et/ou électrostatiques.

Dans un mode de réalisation, la piste conductrice (2) n'est pas configurée pour émettre des signaux tels que des signaux électromagnétiques et/ou électrostatiques.

Dans un mode de réalisation, la résistance couplée à la piste conductrice (2) simule une impédance de contact entre la peau et au moins un moyen de mesure bioélectrique (4).

Dans un mode de réalisation où le au moins un moyen de mesure bioélectrique 4 est une électrode sèche EEG, l'ordre de grandeur de l'impédance du contact entre la peau de l'utilisateur et l'électrode sèche EEG est compris entre 100 kOhms et 10 MOhms (10 Mégaohms).

Dans un mode de réalisation, l'impédance de ladite résistance 8 est comprise entre 10 kOhms et 100 MOhms. Dans un mode de réalisation préférentiel, l'impédance de ladite résistance 8 est comprise entre 100 kOhms et 10 MOhms, préférentiellement comprise entre 500 kOhms et 5 MOhms, très préférentiellement comprise entre 700 kOhms et 2 MOhms, encore plus préférentiellement comprise entre 800 kOhms et 1,5 MOhms.

Dans un mode de réalisation illustré sur la **figure 1** et la **figure 2****,** la résistance 8 fait partie du dispositif de conversion analogique-numérique 6. Dans un autre, mode de réalisation illustré **figure 5****,** la résistance 8 n'est pas intégrée au dispositif de conversion analogique-numérique 6 mais est fixée sur le substrat 5, au contact de la piste conductrice 2. Dans un mode de réalisation non représenté, la résistance n'est pas fixée sur le dispositif de conversion analogique-numérique 6 mais est intégrée sur un circuit imprimé comprenant le dispositif de conversion analogique-numérique 6.

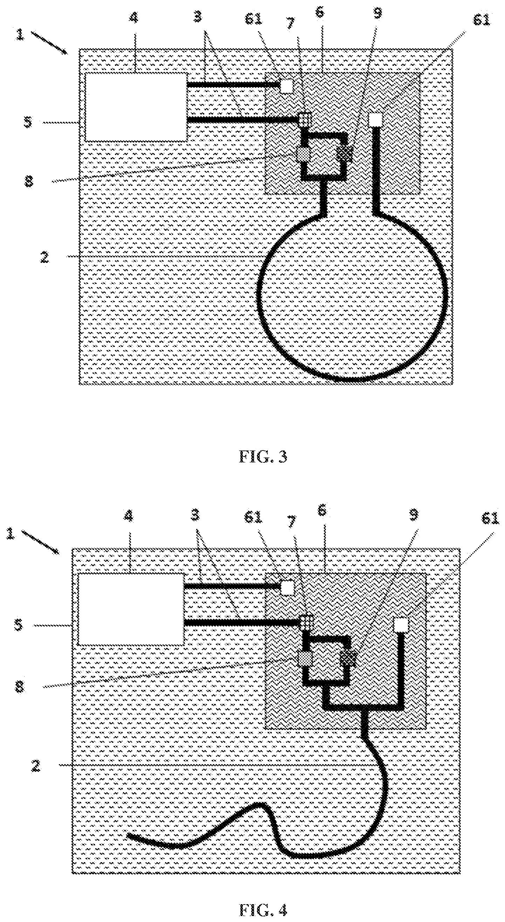

Dans un mode de réalisation illustré **figure 3** et **figure 4****,** pour se rapprocher de l'impédance de contact entre la peau et le au moins un moyen de mesure bioélectrique, le système 1 comprend un condensateur 9 monté en parallèle avec la résistance 8. Ce condensateur 9 permet de modéliser la capacité parasite entre la peau et le au moins un moyen de mesure bioélectrique 4. La capacité du condensateur 9 doit être de même ordre de grandeur, et préférentiellement la plus proche possible de la capacité parasite du contact entre la peau et le au moins un moyen de mesure bioélectrique 4. Ainsi, les signaux électriques mesurés par la piste conductrice 2 peuvent être comparés aux signaux mesurés par le au moins un moyen de mesure bioélectrique 4. L'ordre de grandeur de la capacité dudit condensateur 9 est variable en fonction du moyen de mesure bioélectrique 4 utilisé. Dans un mode de réalisation, ledit condensateur 9 a une capacité comprise entre 10 picoFarad et 100 nanofarad.

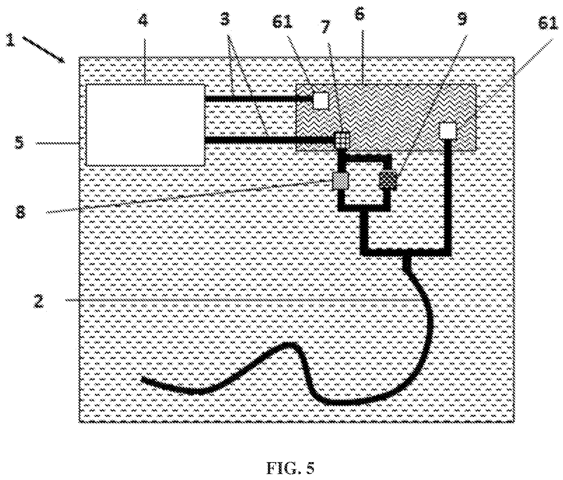

Dans un autre mode de réalisation illustré **figure 5****,** la résistance 8 et le condensateur 9 ne sont pas intégrés au dispositif de conversion analogique-numérique 6 mais sont fixés sur le substrat 5, au contact de la piste conductrice 2. Dans un mode de réalisation non représenté, la résistance 8 et le condensateur 9 ne sont pas intégrés au dispositif de conversion analogique-numérique 6 mais sont intégrés sur un circuit imprimé comprenant le dispositif de conversion analogique-numérique 6.

Le système 1 comprend un substrat 5 textile sur lequel sont montés le moyen de mesure bioélectrique 4, le dispositif de conversion analogique-numérique 6, et la piste conductrice 2. Le système 1 comprend un substrat 5 textile, et ledit moyen de mesure biométrique 4 et ladite piste conductrice 2 sont connectés mécaniquement audit substrat 5 textile Ce substrat 5 permet d'assurer la cohésion des différents composants du système 1. Dans un mode de réalisation, le convertisseur analogique-numérique est également connecté mécaniquement au substrat 5.

Dans un mode de réalisation alternatif non revendiqué, le substrat 5 est un substrat 5 mécanique. Le substrat 5 mécanique peut consister en une plaque de matériau plastique, ou plus généralement de tout support comprenant une surface de contact isolante avec la piste conductrice 2. Dans un mode de réalisation, le substrat 5 mécanique est une armature mécanique qui peut être utilisée pour la fabrication d'un casque. Le substrat 5 mécanique peut être rigide ou flexible.

Le substrat 5 est un substrat 5 textile. Dans un mode de réalisation, le substrat 5 textile est un textile tissé ou tricoté avec des fils isolants.

Le substrat 5 est un substrat 5 textile. L'utilisation d'un substrat 5 textile permet de donner au système 1 ses propriétés de flexibilité et d'élasticité. Dans ce même mode de réalisation, la piste conductrice 2 est réalisée de manière à avoir sensiblement la même flexibilité que le substrat 5 textile. Dans un mode de réalisation où le substrat 5 est un substrat 5 textile, et la piste conductrice 2 comprend au moins un fil conducteur tissé, brodé, tricoté, ou inséré à travers le substrat 5 textile. Dans ce même mode de réalisation, le au moins un fil conducteur est constitué d'un matériau conducteur ou d'au moins un fil textile recouvert d'une surface conductrice, préférentiellement recouvert d'un métal conducteur comme de l'argent. Dans un mode de réalisation alternatif, la piste conductrice 2 est une encre conductrice ou une peinture conductrice imprimée sur le substrat 5 textile.

Dans un mode de réalisation, la piste conductrice 2 est délimitée par ses extrémités. Dans un mode de réalisation, la piste conductrice 2 est délimitée par sa jonction avec le dispositif de conversion analogique-numérique 6. Dans un autre mode de réalisation, la piste conductrice 2 est délimitée par sa jonction avec la résistance, l'entrée 61 du dispositif de conversion analogique-numérique 6 et optionnellement le condensateur 9. Dans un mode de réalisation, la piste conductrice 2 est délimitée par le point de connexion avec le circuit imprimé.

Dans un mode de réalisation, le système 1 comprend plusieurs pistes conductrices 2.

La masse 7 est la branche de référence des potentiels électriques. La masse 7 peut être située sur le dispositif de conversion analogique-numérique 6 ou ailleurs sur le système 1 selon la présente invention, par exemple sur le substrat 5.

L'invention concerne également un vêtement comprenant au moins un système de mesure des perturbations 1 selon la présente invention. Par vêtement, on entend tout textile apte à être porté par un sujet, y compris un bonnet. Dans un mode de réalisation préférentiel, ledit vêtement est destiné à être porté au contact de la peau, de manière à faciliter la fiabilité de la mesure enregistrée par le au moins un moyen de mesure bioélectrique 4, tel qu'un sous-vêtement, un bonnet, un maillot ou un T-shirt.

Dans un mode de réalisation, l'invention est appliquée à un dispositif de mesure déjà existant tel un casque EEG ou autre.

Dans un mode de réalisation, le vêtement est un bonnet qui comprend deux systèmes 1 selon la présente invention. Dans ce mode de réalisation, les deux systèmes 1 selon la présente invention sont disposés de part et d'autre du bonnet. L'utilisateur peut donc disposer un système 1 au contact du côté gauche de son crâne et un système 1 au contact du côté droit de son crâne.

Selon un mode de réalisation, le vêtement selon la présente invention permet de mesurer les perturbations électromagnétiques et/ou électrostatiques extérieures auxquelles est exposé le au moins un moyen de mesure bioélectrique 4.

L'invention concerne également une méthode d'utilisation du système de mesure des perturbations 1 selon la présente invention comprenant les étapes suivantes :
- la mesure du signal obtenu par le au moins un moyen de mesure bioélectrique 4 ;
- la mesure du signal obtenu par la au moins une piste conductrice 2 ; et
- le traitement desdits signaux de manière à retirer, du signal mesuré par le au moins un moyen de mesure bioélectrique, le signal mesuré par la au moins une piste conductrice 2.

L'étape de traitement desdits signaux peut être réalisée par un algorithme, un module électronique, un système de traitement des données ou par un technicien.

### BRÈVE DESCRIPTION DES FIGURES

La **Figure 1** est une vue schématique du système 1 selon un mode de réalisation de la présente invention où la piste conductrice 2 forme une antenne boucle. La piste conductrice 2 comprend deux extrémités, une première extrémité est connectée à l'entrée 61 du dispositif de conversion analogique-numérique 6 et une seconde extrémité est connectée à la masse 7 du système 1.
La **Figure 2** est une vue schématique du système 1 selon un mode de réalisation de la présente invention où la piste conductrice 2 ne forme pas un circuit fermé. La piste conductrice 2 comprend une première extrémité connectée à l'entrée 61 du dispositif de conversion analogique-numérique 6 et connectée à la masse 7 du système 1. La piste conductrice 2 comprend une deuxième extrémité qui n'est reliée à aucune résistance ni aucun élément électriquement conducteur, de manière à créer un circuit ouvert.
La **Figure 3** est une vue schématique du système 1 selon un mode de réalisation de la présente invention selon la Figure 1, dans lequel un condensateur 9 est monté en parallèle de la résistance 8.
La **Figure 4** est une vue schématique du système 1 selon un mode de réalisation de la présente invention selon la Figure 2, dans lequel un condensateur 9 est monté en parallèle de la résistance 8.
La **Figure 5** est une vue schématique du système 1 selon un mode de réalisation de la présente invention selon la Figure 4, dans lequel la résistance 8 et le condensateur 9 sont montés sur le substrat 5 textile ou mécanique et non sur le dispositif de conversion analogique-numérique 6.
Les figures ont été réalisées dans un but pédagogique, afin de permettre au lecteur de la présente demande de brevet de mieux cerner l'invention. Les figures ne sont donc pas à l'échelle et la portée de ladite demande de brevet ne pourrait se voir limitée par les distances mesurées sur les figures.

### RÉFÉRENCES

1 - Système de mesure des perturbations ;
2 - Piste conductrice ;
3 - Moyen de connexion électrique :
4 - Moyen de mesure bioélectrique :
5 - Substrat ;
6 - Dispositif de conversion analogique-numérique ;
61 - Entrée du dispositif de conversion analogique-numérique ;
7 - Masse du système ;
8 - Résistance ;
9 - Condensateur.

## Revendications

1. Système de mesure des perturbations (1) apte à être intégré dans un vêtement destiné à être porté par un utilisateur, ledit système comprenant :
- au moins un moyen de mesure bioélectrique (4) pour mesurer un signal électrique corporel et susceptible de recevoir des perturbations électromagnétiques et/ou électrostatiques extérieures ;
- un dispositif de conversion analogique-numérique (6) connecté électriquement audit au moins un moyen de mesure bioélectrique (4) ; et
- au moins une piste conductrice (2) de mesure de signaux électromagnétiques et/ou électrostatiques, ladite piste conductrice (2) étant connectée électriquement à une masse (7) du système (1) par l'intermédiaire d'une résistance (8) et à une entrée (61) du dispositif de conversion analogique-numérique (6), ladite piste conductrice (2) ayant une longueur comprise entre 5 cm et 500 cm pour permettre la mesure des perturbations électromagnétiques et/ou électrostatiques extérieures susceptibles de perturber le signal mesuré ; et
- un substrat (5) textile dans lequel ledit moyen de mesure biométrique (4) et ladite au moins une piste conductrice (2) sont connectés mécaniquement audit substrat (5) textile.

2. Système (1) selon la revendication **1, caractérisé en ce qu'**il est configuré pour que le signal électrique mesuré par là au moins une piste conductrice (2) est soustrait au signal électrique mesuré par le au moins un moyen de mesure bioélectrique (4) pour produire un signal résultant épuré des signaux extérieurs parasites.

3. Système (1) selon la revendication **1** ou la revendication **2, caractérisé en ce qu'**il est configuré pour qu'une résistance couplée à la piste conductrice (2) simule une impédance de contact entre la peau et au moins un moyen de mesure bioélectrique (4).

4. Système (1) selon l'une quelconque des revendications **1** à **3,** dans lequel l'impédance de ladite résistance (8) est comprise entre 10 kOhms et 100 MOhms.

5. Système (1) selon l'une quelconque des revendications **1** à **4,** comprenant en outre un condensateur (9) connecté en parallèle de ladite résistance (8).

6. Système (1) selon la revendication **5,** dans lequel la capacité dudit condensateur (9) est comprise entre de 10 picofarad et 100 nanofarad.

7. Système (1) selon l'une quelconque des revendications **1** à **6,** dans lequel la au moins une piste conductrice (2) comprend une première extrémité connectée électriquement à la masse (7) du système (1) et à une entrée (61) du dispositif de conversion analogique-numérique (6), et une deuxième extrémité libre.

8. Système (1) selon l'une quelconque des revendications **1** à **6,** dans lequel la au moins une piste conductrice (2) comprend une première extrémité connectée électriquement à la masse (7) du système (1) et une deuxième extrémité connectée électriquement à une entrée (61) du dispositif de conversion analogique-numérique (6).

9. Système (1) selon la revendication 8, dans lequel le substrat (5) est un substrat (5) textile et là au moins une piste conductrice (2) :
- comprend au moins un fil conducteur tissé, brodé, tricoté ou inséré à travers le substrat (5) textile ; ou
- est une encre conductrice ou une peinture conductrice imprimée sur le substrat (5) textile.

10. Vêtement comprenant au moins un système de mesure des perturbations (1) selon l'une quelconque des revendications **1** à **9.**

11. Méthode d'utilisation du système de mesure des perturbations (1) selon l'une quelconque des revendications **1** à **9** ou du vêtement selon la revendication **11** comprenant les étapes suivantes :
- la mesure du signal corporel obtenu par le au moins un moyen de mesure bioélectrique (4) ;
- la mesure du signal des perturbations électromagnétiques et/ou électrostatiques, obtenu par la au moins une piste conductrice (2) ; et
- le traitement desdits signaux de manière à retirer, du signal mesuré par le au moins un moyen de mesure bioélectrique, le signal mesuré par la au moins une piste conductrice (2).

## Claims

1. Disturbance measurement system (1) capable of being incorporated in a garment intended to be worn by a user, said system comprising:
- at least one bioelectrical measurement means (4) for measuring a body electrical signal and likely of receiving external electromagnetic and/or electrostatic disturbances;
- an analogue-to-digital conversion device (6) electrically connected to said at least one bioelectrical measurement means (4); and
- at least one conductive track (2) for measuring electromagnetic and/or electrostatic signals, said conductive track (2) being electrically connected to a ground (7) of the system (1) via a resistor (8) and to an input (61) of the analogue-to-digital conversion device (6), said conductive track (2) having a length between 5 cm and 500 cm to enable the measurement of external electromagnetic and/or electrostatic disturbances likely to disturb the measured signal; and
- a textile substrate (5), wherein said biometric measurement means (4) and said at least one conductive track (2) are mechanically connected to said textile substrate (5).

2. System (1) according to claim **1, characterised in that** it is configured so that the electrical signal measured by the at least one conductive track (2) is subtracted from the electrical signal measured by the at least one bioelectrical measurement means (4) to produce a resulting signal purified from the parasitic external signals.

3. System (1) according to claim **1** or claim **2, characterised in that** it is configured for a resistor coupled to the conductive track (2) to simulate a contact impedance between the skin and at least one bioelectric measurement means (4).

4. System (1) according to any one of claims **1** to **3,** wherein the impedance of said resistor (8) is between 10 kOhms and 100 MOhms.

5. System (1) according to any one of claims **1** to **4,** further comprising a capacitor (9) connected in parallel to said resistor (8).

6. System (1) according to claim **5,** wherein the capacitance of said capacitor (9) is between 10 picofarad and 100 nanofarad.

7. System (1) according to any one of claims **1** to **6,** wherein the at least one conductive track (2) comprises a first end electrically connected to the ground (7) of the system (1) and to an input (61) of the analogue-to-digital conversion device (6), and a free second end.

8. System (1) according to any one of claims **1** to **6,** wherein the at least one conductive track (2) comprises a first end electrically connected to the ground (7) of the system (1) and a second end electrically connected to an input (61) of the analogue-to-digital conversion device (6).

9. System (1) according to claim 8, wherein the substrate (5) is a textile substrate (5) and the at least one conductive track (2):
- comprises at least one conductive yarn woven, embroidered, knitted or inserted through the textile substrate (5); or
- is a conductive ink or conductive paint printed on the textile substrate (5).

10. Garment comprising at least one disturbance measurement system (1) according to any one of claims **1** to **9.**

11. Method for using the disturbance measurement system (1) according to any one of claims **1** to **9** or for using the garment according to claim **11,** comprising the following steps:
- measuring the body signal obtained by the at least one bioelectric measurement means (4);
- measuring the signal of electromagnetic and/or electrostatic disturbances, obtained by the at least one conductive track (2); and
- processing said signals so as to remove, from the signal measured by the at least one bioelectric measurement means, the signal measured by the at least one conductive track (2).

## Patentansprüche

1. Störungsmesssystem (1), das in ein Kleidungsstück integriert werden kann, das von einem Benutzer getragen werden soll, wobei das System Folgendes umfasst:
- mindestens ein bioelektrisches Messmittel (4) zum Messen eines elektrischen Körpersignals, das externe elektromagnetische und/oder elektrostatische Störungen empfangen werden kann;
- eine Analog-Digital-Umwandlungsvorrichtung (6), die elektrisch mit dem mindestens einen bioelektrischen Messmittel (4) verbunden ist; und
- mindestens eine Leiterbahn (2) zum Messen elektromagnetischer und/oder elektrostatischer Signale, wobei die Leiterbahn (2) über einen Widerstand (8) mit einer Masse (7) des Systems (1) und einem Eingang (61) der Analog-Digital-Umwandlungsvorrichtung (6) elektrisch verbunden ist, wobei die Leiterbahn (2) eine Länge zwischen 5 cm und 500 cm aufweist, um das Messen externer elektromagnetischer und/oder elektrostatischer Störungen zu ermöglichen, die das gemessene Signal stören könnten; und
- ein textiles Substrat (5), wobei das biometrische Messmittel (4) und die mindestens eine Leiterbahn (2) mechanisch mit dem textilen Substrat (5) verbunden sind.

2. System (1) nach Anspruch **1, dadurch gekennzeichnet, dass** es so eingerichtet ist, dass das dort von mindestens einer Leiterbahn (2) gemessene elektrische Signal von dem von dem mindestens einen bioelektrischen Messmittel (4) gemessenen elektrischen Signal subtrahiert wird, um ein von externen Störsignalen bereinigtes resultierendes Signal zu erzeugen.

3. System (1) nach Anspruch **1** oder Anspruch **2, dadurch gekennzeichnet, dass** es so eingerichtet ist, dass ein mit der Leiterbahn (2) gekoppelter Widerstand eine Kontaktimpedanz zwischen der Haut und mindestens einem bioelektrischen Messmittel (4) simuliert.

4. System (1) nach einem der Ansprüche **1** bis **3,** wobei die Impedanz des Widerstands (8) zwischen 10 kOhm und 100 MOhm liegt.

5. System (1) nach einem der Ansprüche **1** bis **4,** ferner umfassend einen Kondensator (9), der parallel zu dem Widerstand (8) geschaltet ist.

6. System (1) nach Anspruch **5,** wobei die Kapazität des Kondensators (9) zwischen 10 Picofarad und 100 Nanofarad liegt.

7. System (1) nach einem der Ansprüche **1** bis **6,** wobei die mindestens eine Leiterbahn (2) ein erstes Ende umfasst, das elektrisch mit der Masse (7) des Systems (1) und einem Eingang (61) der Analog-Digital-Umwandlungsvorrichtung (6) verbunden ist, und ein zweites freies Ende.

8. System (1) nach einem der Ansprüche **1** bis **6,** wobei die mindestens eine Leiterbahn (2) ein erstes Ende umfasst, das elektrisch mit der Masse (7) des Systems (1) verbunden ist, und ein zweites Ende, das elektrisch mit einem Eingang (61) der Analog-Digital-Umwandlungsvorrichtung (6) verbunden ist.

9. System (1) nach Anspruch 8, wobei das Substrat (5) ein textiles Substrat (5) ist und dort mindestens eine Leiterbahn (2):
- mindestens einen leitfähigen Faden umfasst, der in das textile Substrat (5) eingewebt, eingestickt, eingestrickt oder eingefügt ist; oder
- eine leitfähige Tinte oder eine leitfähige Farbe ist, die auf das textile Substrat (5) gedruckt ist.

10. Kleidungsstück, das mindestens ein Störungsmesssystem (1) nach einem der Ansprüche **1** bis **9** umfasst.

11. Verfahren zur Verwendung des Störungsmesssystems (1) nach einem der Ansprüche **1** bis **9** oder des Kleidungsstücks nach Anspruch **11,** umfassend die folgenden Schritte:
- Messen des durch das mindestens eine bioelektrische Messmittel (4) erhaltenen Körpersignals;
- Messen des Signals der elektromagnetischen und/oder elektrostatischen Störungen, das von der mindestens einen Leiterbahn (2) erhalten wird; und
- Verarbeiten der Signale, derart, dass aus dem von dem mindestens einen bioelektrischen Messmittel gemessenen Signal das von der mindestens einen Leiterbahn (2) gemessene Signal entnommen wird.
